# EUROPEAN PATENT APPLICATION

(11) **EP 2 415 427 A1**
(43) Date of publication of application: **08.02.2012**
(21) Application number: 09842689.3
(22) Date of filing: 18.09.2009
(51) Int. Cl.: A61F 13/15, A61F 13/472, A61F 13/56

(54) **ABSORBENT ARTICLE**

(30) Priority: 31.03.2009 JP 2009087196
(71) Applicant: Unicharm Corporation, Ehime 799-0111 (JP)
(72) Inventor: NOMOTO, Takashi, Kanonji-shi Kagawa 769-1602 (JP); NAGATA, Akiyasu, Kanonji-shi Kagawa 769-1602 (JP)
(74) Representative: Peter, Julian
(86) International application number: PCT/JP2009/066844
(87) International publication number: WO 2010/113337

(57) **Abstract**

A sanitary napkin (1) comprises an absorbing body (2). The absorbing body (2) includes a fluid-permeable top sheet (3), a fluid-impermeable back sheet (4), and an absorber (5) arranged between the top sheet (3) and the back sheet (4). The napkin comprises a cover sheet (8) for covering the surface of the top sheet (3). A circumferential edge region (8P) of the cover sheet (8) is joined to the absorbing body (2). A plurality of flaps for fixing the absorbing body (2) to underwear are sectionalized by a sectionalizing line (9) in the central region (8C) of the cover sheet. At the time of use, the flaps are folded in the folding regions and are expanded so as to be fixed to the underwear. The flaps include a front flap (10F), a rear flap (10B), a left flap (10L) and a right flap (10R). A leakage-preventing region is formed on the cover sheet (8) between the folding regions and the circumferential edge region (8P).

## Description

### Technical Field

This invention relates to an absorbent article.

### Background Art

There has been known an absorbent article having an absorbing body. The absorbing body includes a fluid-permeable top sheet, a fluid-impermeable back sheet, and an absorber arranged between the top sheet and the back sheet. A fluid-impermeable belt-like sheet is attached to the front edge, rear edge, left side edge and right side edge of the absorbing body. A pocket is formed by utilizing a belt-like sheet portion that rises from the surface of the top sheet so as to temporarily stop the absorbed fluid (see PTL1). Namely, in this absorbent article, the belt-like sheet portion rising from the surface of the top sheet works as a leakage-preventing wall.

### Citation List

Patent Literature
   PTZ1 JP-UM-A-5-91633

### Summary of Invention

### Technical Problem

However, in the above absorbent article, the belt-like sheet is present on only the circumferential portion of the absorbing body, and therefore the top sheet remains exposed. Therefore, the top sheet may become fouled while the absorbent article is being handled, which is hygienically undesirable.

### Solution to Problems

In order to solve the above problem according to the present invention, there is provided an absorbent article comprising an absorbing body, the absorbing body including a fluid-permeable top sheet, a fluid-impermeable back sheet, and an absorber arranged between the top sheet and the back sheet, wherein the absorbent article further comprises a cover sheet for covering the surface of the top sheet, wherein a circumferential edge region of the cover sheet is joined to the absorbing body, and, in a central region of the cover sheet, a plurality of flaps for fixing the absorbing body to clothing are sectionalized by a sectionalizing line which is constituted by a weakened line or a cut line, wherein the flaps include a front flap and a rear flap that are folded in the folding regions along the front and rear edges of the absorbing body and are expanded in the back-and-forth direction, and a left flap and a right flap that are folded in the folding regions along the left and right side edges of the absorbing body and are expanded in the right-and-left direction and wherein, at the time of use, the flaps are folded in the folding regions and are expanded so as to be fixed to the clothing, and wherein a leakage-preventing region is formed on the cover sheet between the folding regions and the circumferential edge region.

### Advantageous Effect of Invention

The top sheet can be protected while securing the effect for preventing the leakage.

### Brief Description of Drawings

Fig. 1 is a front view of a sanitary napkin.
Fig. 2 is a back view of the sanitary napkin.
Fig. 3 is a schematic transverse sectional view of the sanitary napkin along the line III-III in Fig. 1.
Fig. 4 is a front view of the sanitary napkin with the flaps being expanded.
Fig. 5 is a schematic longitudinal sectional view of the sanitary napkin illustrating the state of use.
Fig. 6 is a schematic transverse sectional view of the sanitary napkin illustrating the state of use.
Fig. 7 is a front view of the sanitary napkin according to another embodiment of the invention.
Fig. 8A is a front view of the sanitary napkin according to a further embodiment of the invention.
Fig. 8B is a schematic transverse sectional view of the sanitary napkin according to a further embodiment of the invention.
Fig. 9 is a front view of the sanitary napkin showing an alternative embodiment of the sectionalizing lines.
Fig. 10 is a front view of the sanitary napkin showing an alternative embodiment of the cover sheet.

### Description of Embodiments

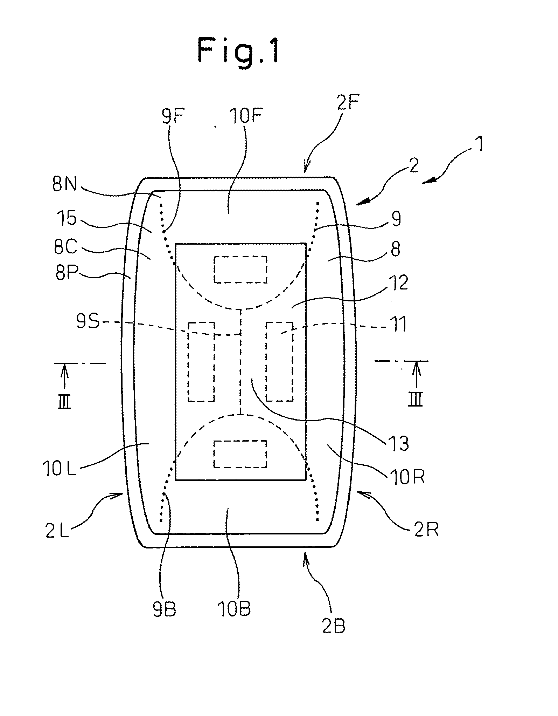

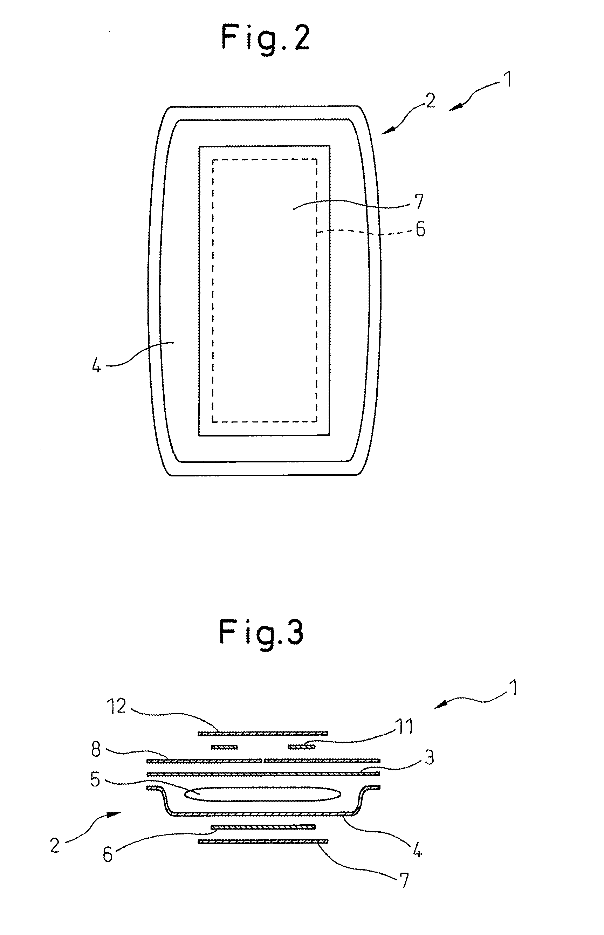

Figs. 1 to 3 illustrate a case where the invention is applied to a sanitary napkin. However, the invention can be, further, applied to other absorbent articles such as a panty liner, incontinence pad and the like.

Referring to Figs. 1 to 3, the sanitary napkin (hereinafter also referred to simply as "napkin") 1 according to the embodiment of the invention includes an absorbing body 2. The absorbing body 2 includes a fluid-permeable top sheet 3, a fluid-impermeable back sheet 4 and an absorber 5 capable of holding fluid being arranged between the top sheet 3 and the back sheet 4. The top sheet 3 and the back sheet 4 are substantially of the same size, and are joined together along the circumferential edges thereof by hot-melt adhesion or heat-sealing. Further, a sticking portion 6 is joined to the surface of the back sheet 4 that, in use, faces the clothing such as underwear so that the absorbing body 2 can be fixed to the underwear. The sticking portion 6 is covered with a protection sheet 7.

In Fig. 1, reference numerals 2F, 2B, 2L and 2R denote a front edge, a rear edge, a left side edge and a right side edge of the napkin 1 or the absorbing body 2. The front, rear, left and right are corresponding to the front, rear, left and right of the body of the user.

The surface of the top sheet 3 that comes in contact with the user's skin at the time of use is covered with a fluid-impermeable cover sheet 8. The cover sheet 8 is substantially of the same size as the absorbing body 2, and therefore covers substantially the entire surface of the top sheet 3. Further, the cover sheet 8 is joined to the top sheet 3 or the absorbing body 2 along its annular circumferential edge region 8P by hot-melt adhesion or heat-sealing. On the other hand, a central region 8C other than the circumferential edge region 8P is not joined to the top sheet 3.

A sectionalizing line 9 such as a perforated weakened line is formed in advance in the central region 8C of the cover sheet 8, and a plurality of flaps are sectionalized or formed in the central region 8C by the sectionalizing line 9 to fix the absorbing body 2 to the underwear. The sectionalizing line 9 may be constituted by a cut line that completely cuts the cover sheet 8.

In the embodiment of the invention, the sectionalizing line 9 is constituted by a U-shaped curved portion 9F that expands toward the front edge 2F, a U-shaped curved portion 9B that expands toward the rear edge 2B, and a linear portion 9S that connects together the vertexes of the curved portions 9F and 9B substantially at the center of the cover sheet 8. Therefore, in the central region 8C, there are sectionalized four flaps, i.e., a semi-elliptical front flap 10F and rear flap 10B having curved ends, and a trapezoidal left flap 10L and right flap 10R. In this case, the front and rear flaps 10F and 10B are positioned symmetrically to each other, and the left and right flaps 10L and 10R are positioned symmetrically to each other, too.

The sectionalizing line 9, in this case, does not reach the circumferential edge region 8P, and therefore non-cut regions 8N are formed between the ends of the sectionalizing line 9 and the circumferential edge region 8P.

Sticking portions 11 are joined to the surfaces of these flaps 10F, 10B, 10L and 10R to fix the corresponding flaps 10F, 10B, 10L and 10R to the underwear. The sticking portions 11 are covered with a common protection sheet 12. In this case, the sticking portions 11 are joined to the flaps 10F, 10B, 10L and 10R being separated away from the sectionalizing line 9. Therefore, non-sticking regions 13 are formed on the flaps 10F, 10B, 10L and 10R surrounding the sticking portions 11.

Next, materials of the elements will be described.

The top sheet 3 is constituted by, for example, a porous or nonporous nonwoven fabric or a porous plastic sheet.

The back sheet 4 is constituted by, for example, a hydrophobic nonwoven fabric, a water-impermeable plastic film, a laminated sheet of a nonwoven fabric and a water-impermeable plastic film, a highly water resistant melt-blown nonwoven fabric, or an SMS nonwoven fabric sandwiched by spun-bonded nonwoven fabrics having a large strength.

The absorber 5 is constituted by, for example, a fluffy pulp or an air-laid nonwoven fabric and a highly absorbent polymer. The fluffy pulp is constituted by an artificial cellulose fiber such as chemical pulp, cellulose fiber, rayon or acetate, while the air-laid nonwoven fabric is constituted by a nonwoven fabric obtained by, for example, melt-adhering the pulp and the synthetic fiber together or by fixing them together with a binder, and the highly absorbent polymer is constituted by, for example, a granular or fibrous polymer of the type of starch, acrylic acid or amino acid.

The sticking portions 6 and 11 are constituted by a hot-melt adhesive such as styrene/isoprene/styrene block copolymer (SIS), styrene/butadiene/styrene block copolymer (SBS) or styrene/ethylene/butylene/ethylene copolymer (SEBS).

The cover sheet 8 is constituted by, for example, a hydrophobic nonwoven fabric, a water-impermeable plastic film, a laminated sheet of a nonwoven fabric and a water-impermeable plastic film, a highly water resistant melt-blown nonwoven fabric, or an SMS nonwoven fabric sandwiched by spun-bonded nonwoven fabrics having a large strength, and is, preferably, constituted by the hydrophobic nonwoven fabric. The basis weight of the cover sheet 8 is, preferably, from 15 g/m² to 60 g/m². When the cover sheet is constituted by using the plastic film, it is desired that the plastic film has a draping length of 20 mm to 100 mm and, more preferably, 30 mm to 70 mm relying on a cantilever method. If the draping length is shorter than 20 mm, the cover sheet 8 may be easily twisted. If the draping length is longer than 100 mm, on the other hand, the cover sheet 8 may become so hard as to deteriorate the feeling of use of the napkin 1 resulting in a decrease in the underwear follow-up performance and permitting the absorbed fluid to leak out.

The above cantilever method is conducted as described below in compliance with the JIS-L1018. Namely, five pieces of the object to be measured having a length of 150 mm and a width of 25 mm are overlapped one upon the other to obtain a measuring sample. Next, by using a cantilever manufactured by Daiei Kagaku Seiki Mfg. Co., the measuring sample is held under a holding plate of the cantilever and is slid in a tilted direction at a speed of 5 mm/sec to automatically measure a distance it has traveled. Measurements are taken in both of the cases of when the surface of the measuring sample faces down and when the back surface of the measuring sample is on the lower side, and an average value is regarded as the measured result.

The napkin 1 is fixed to the underwear as described below. Namely, the protection sheet 7 on the side of the back sheet 4 is removed, first. Next, the napkin 1 or the absorbing body 2 is fixed to the underwear via the sticking portion 6. The top sheet 3 has been covered with the cover sheet 8 and is prevented from being fouled.

Next, the central region 8C of the cover sheet 8 is broken along the sectionalizing line 9. As a result, there are formed four flaps 10F, 10B, 10L and 10R that are separated from each other.

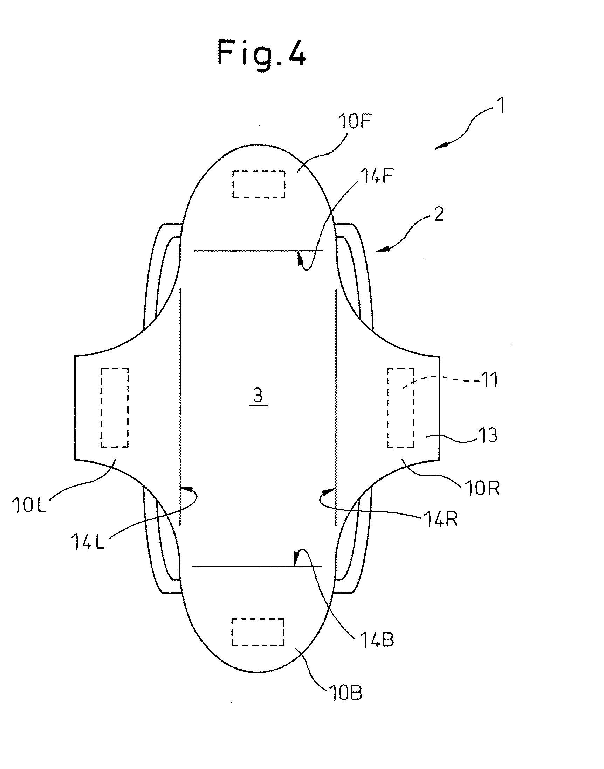

Next, as shown in Fig. 4, these flaps 10F, 10B, 10L and 10R are expanded, respectively. That is, the front flap 10F is expanded forward being folded in a folding region 14F along the front edge 2F, and the rear flap 10B is expanded rearward being folded in a folding region 14B along the rear edge 2B. Further, the left flap 10L is expanded toward the left being folded in a folding region 14L along the left edge 2L, and the right flap 10R is expanded toward the right being folded in a folding region 14R along the right edge 2R. As a result, the top sheet 3 is exposed.

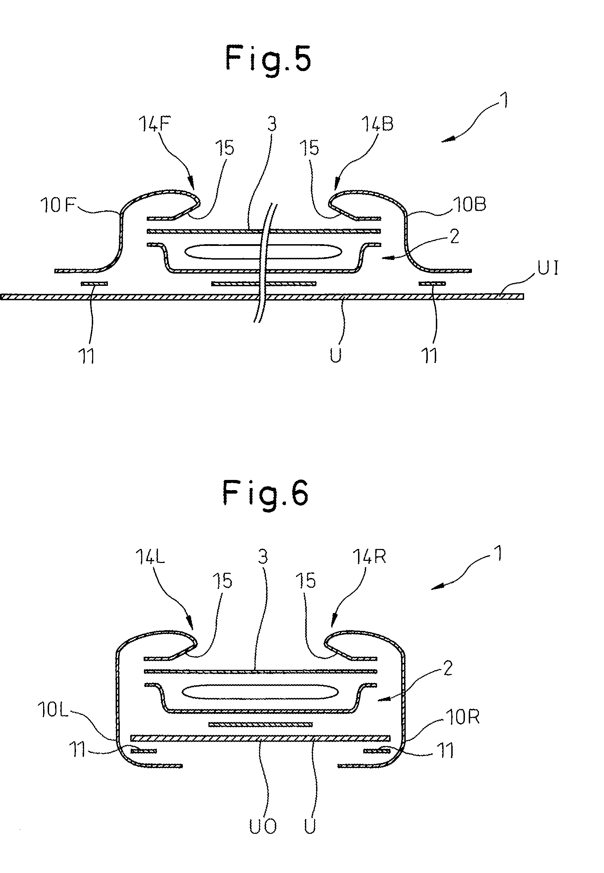

Next, the flaps 10F, 10B, 10L and 10R are fixed to the underwear via the respective sticking portions 11. That is, as shown in Fig. 5 which is a longitudinal sectional view of the napkin 1, the front flap 10F is fixed to the inner surface UI of the underwear U in front of the absorbing body 2, and the rear flap 10B is fixed to the inner surface UI of the underwear U at the back of the absorbing body 2. As shown in Fig. 6 which is a transverse sectional view of the napkin 1, further, the left flap 10L and the right flap 10R are fixed to the outer surface UO of the underwear U under the absorbing body 2.

Thus, the flaps 10F, 10B, 10L and 10R are fixed to the underwear U and, therefore, the absorbing body 2 is fixed to the underwear U. In this case, the absorbing body 2 is fixed to the underwear U by both the flaps in the back-and-forth direction that are fixed being expanded in the back-and-forth direction like the front flap 10F and the rear flap 10B, and the flaps in the right-and-left direction that are fixed being expanded in the right-and-left direction like the left flap 10L and the right flap 10R. Further, the flaps fixed to the inner surface UI of the underwear like the front flap 10F and the rear flap 10B, are held sandwiched between the underwear U and the body of the user. On the other hand, the flaps fixed to the outer surface UO of the underwear U like the left flap 10L and the right flap 10R, work to hold the underwear U between these flaps and the absorbing body 2.

Despite external forces exerted on the absorbing body 2 from various directions, the absorbing body 2 can be suppressed from being twisted or deviated in position. Namely, the absorbing body 2 can be reliably fixed to the underwear U, and therefore the absorbed fluid is suppressed from leaking. In addition, the user is liberated from the anxiety of leakage.

According to the embodiment of the invention, the front ends of the flaps fixed to the inner surface like the front flap 10F and the rear flap 10B are curved, suppressing pain or offensive feeling that may be caused when the front flap 10F and the rear flap 10B come in contact with the body of the user.

Further, non-sticking regions 13 are formed on the flaps 10F, 10B, 10L and 10R or, concretely, at their end portions, enabling the user to handle the flaps 10F, 10B, 10L and 10R by picking up the non-sticking regions 13. Therefore, it is, allowed to easily fix the absorbing body 2 to the underwear U.

As will be understood from Fig. 4, the folding regions 14F, 14B, 14L and 14R are positioned on straight lines connecting the two neighboring ends of the sectionalizing line 9. As shown in Fig. 1, on the other hand, non-cut regions 8N are formed between the ends of the sectionalizing line 9 and the circumferential edge region 8P. As shown in Figs. 5 and 6, therefore, the cover sheet 8 forms a leakage-preventing region 15 between the circumferential edge region 8P and the folded regions 14F, 14B, 14L and 14R or the flaps 10F, 10B, 10L and 10R, the leakage-preventing region 15 continuing over the whole circumference of the cover sheet 8. The leakage-preventing region 15 limits the fluid absorbed by the absorbing body 2 from flowing out, and therefore works as a leakage-preventing wall along the surface of the top sheet 3.

In the embodiment of the invention, a piece of cover sheet 8 is simply joined to the absorbing body 2 to form the leakage-preventing region 15 that works as the leakage-preventing wall. Therefore, the leakage-preventing action can be easily obtained. The leakage-preventing region 15 has no seam. In addition, if the flaps 10F, 10B, 10L and 10R are pulled for fixing to the underwear U, the inner edge of the leakage-preventing region 15 rises from the top sheet 3, and the absorbed fluid is reliably trapped by the leakage-preventing region 15. This makes it possible to reliably suppress the leakage of the absorbed fluid.

The width of the circumferential edge region 8P is, desirably, 2 mm to 10 mm and, more desirably, 3 mm to 5 mm. This is because if the width of the circumferential edge region 8P is smaller than 2 mm, then the junction strength of the cover sheet 8 and the absorbing body 2 decreases. If the width of the circumferential edge region 8P is larger than 10 mm, the exposed surface of the top sheet 3 becomes small when the flaps 10F, 10B, 10L and 10R are expanded. Namely, if the top sheet is broadly exposed, leakage of the absorbed fluid can be suppressed even if the position where the napkin 1 or the absorbing body 2 is fixed to the underwear U is deviated from the proper position to some extent.

On the other hand, the width of the non-cut regions 8N is, desirably, 2 mm to 10 mm and, more desirably, 3 mm to 5 mm. This is because if the width of the non-cut regions 8N is smaller than 2 mm, the tensile strength of the cover sheet 8 in the non-cut regions 8N decreases, and therefore the leakage-preventing effect of the leakage-preventing region 15 decreases. If the width of the non-cut regions 8N is larger than 10 mm, the exposed surface of the top sheet 3 becomes small when the flaps 10F, 10B, 10L and 10R are expanded.

Next, another embodiment of the invention will be described.

According to another embodiment of the invention, the regions surrounding the sticking portions 11 in the flaps 10F, 10B, 10L and 10R have a rigidity larger than the rigidity of other regions.

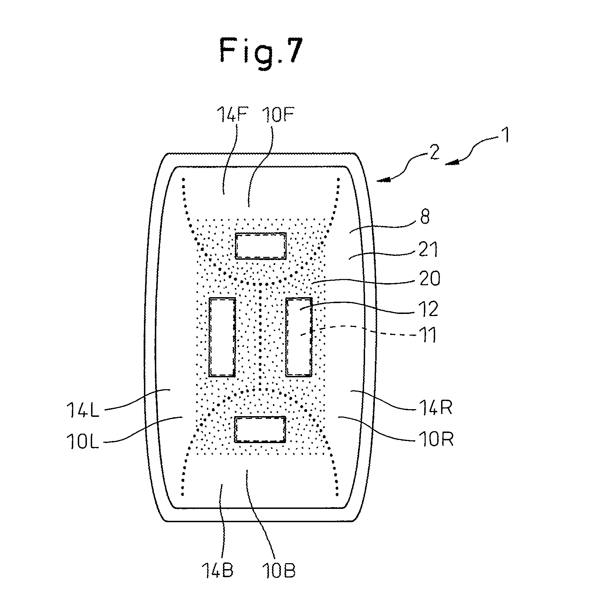

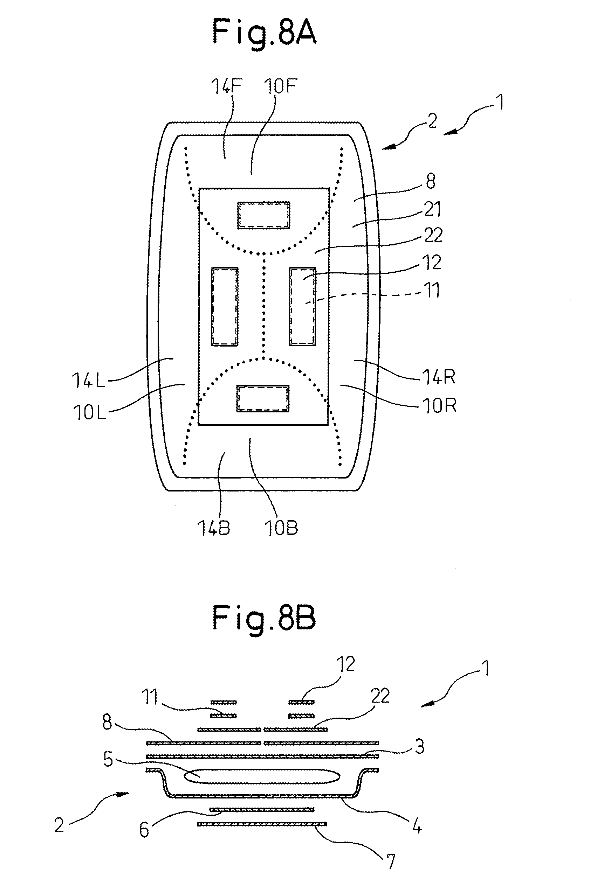

That is, in the embodiment shown in Fig. 7, an embossed region 20 is provided surrounding the sticking portions 11. However, the remaining region 21, is not embossed. In an embodiment shown in Figs. 8(A) and 8(B), on the other hand, an additional sheet 22 is joined surrounding the sticking portions 11. However, the additional sheet 22, is not joined to the remaining region 21.

This makes it easy to handle the flaps 10F, 10B, 10L and 10R. Therefore, the flaps 10F, 10B, 10L and 10R can be easily fixed to the underwear U.

It is desired that the ratio occupied by the embossed region 20 or the additional sheet 22 to the whole of the flaps 10F, 10B, 10L and 10R is not less than 10% and is, particularly, 40% to 100%.

In the embodiment shown in Fig. 7, if the embossed region 20 is of a rugged pattern, the contact area can be decreased between the flaps 10F, 10B and the body of the user, preventing stuffiness and improving feeling. This, further, excels in transferring the hot-melt adhesive of when the sticking portions 11 are joined to the flaps 10F, 10B, 10L and 10R.

In the embodiment shown in Figs. 8(A) and 8(B), further, the additional sheet 22 may be of the same material as the cover sheet 8, or may be of the other material. Further, the additional sheet 22 may be joined to the back surface of the cover sheet 8. A hot-melt adhesive or the like is used for joining the additional sheet 22. A hot-melt adhesive or the like is used for joining the additional sheet 22. The sectionalizing line is formed after the additional sheet 22 is joined to the cover sheet 8 and is, therefore, formed in the additional sheet 22, too.

In the embodiments shown in Fig. 7 and Figs. 8(A) and 8(B), the sticking portions 11 are covered with separate protection sheets 12. However, a common protection sheet 12 may be provided, as a matter of course.

This, in other words, means that in the embodiments shown in Fig. 7 and Figs. 8(A) and 8(B), the remaining region 21 inclusive of the folding regions 14F, 14B, 14L and 14R has a rigidity smaller than the rigidity of the embossed region 20 or the region to where the additional sheet 22 is joined. As a result, the flaps 10F, 10B, 10L and 10R can be easily folded and expanded.

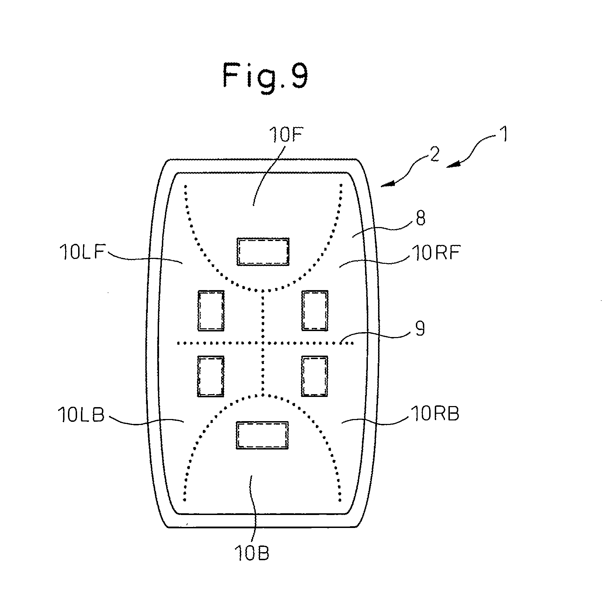

In the foregoing description, the sectionalizing line 9 is so formed that four flaps are sectionalized in the central region 8C of the cover sheet 8. However, the number of the flaps may not have to be four. That is, as shown in Fig. 9, the sectionalizing line 9 may be so formed as to sectionalize six flaps 10F, 10B, 10LF, 10LB, 10RF and 10RB in the central region 8C.

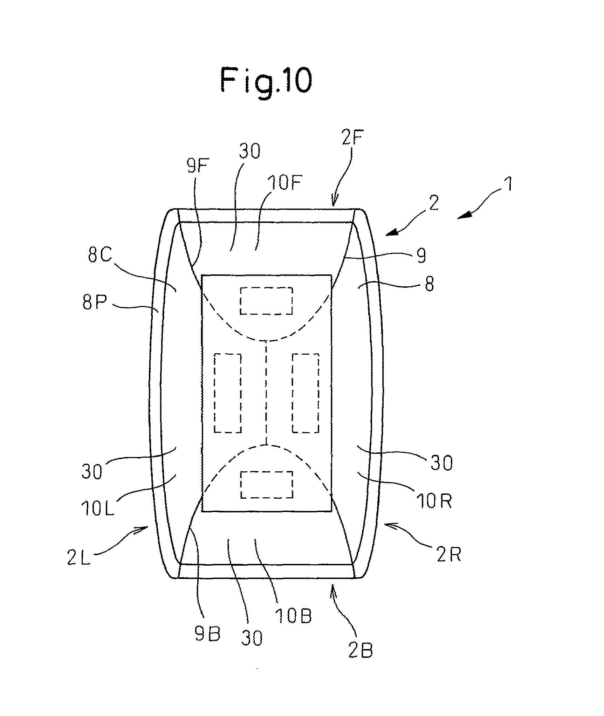

In the foregoing description, further, the cover sheet 8 is constituted by a piece of sheet. However, the cover sheet 8 can be constituted by a plurality of pieces of separate cover sheet portions. Namely, in an embodiment shown in Fig. 10, the cover sheet 8 is constituted by, for example, four pieces of cover sheet portions 30. In this case, the cover sheet portions 30 cover portions of the surface of the top sheet 3; i.e., the cover sheet portions 30 substantially cover the entire surface of the top sheet 3. Further, the sectionalizing lines 9 formed among the neighboring cover sheet portions 30 are extending traversing the circumferential edge region 8P. In the embodiment shown in Fig. 10, further, the cover sheet portions 30 are forming the flaps 10F, 10B, 10L and 10R, respectively. However, the plurality of flaps 10F, 10B, 10L and 10R may be formed by a piece of cover sheet portion 30. Further, the cover sheet portions 30 may be overlapped one upon the other.

### Reference Signs List

- 1: sanitary napkin
- 2: absorbing body
- 3: top sheet
- 4: back sheet
- 5: absorber
- 8: cover sheet
- 8C: central region
- 8P: circumferential edge region
- 9: sectionalizing line
- 10F: front flap
- 10B: rear flap
- 10L: left flap
- 10R: right flap
- 11: sticking portions
- 13: non-sticking portions
- 14F, 14B, 14L, 14R: folding regions
- 15: leakage-preventing region
- 20: embossed region
- 21: remaining region
- 22: additional sheet

## Claims

1. An absorbent article comprising an absorbing body, the absorbing body including a fluid-permeable top sheet, a fluid-impermeable back sheet, and an absorber arranged between the top sheet and the back sheet,
wherein the absorbent article further comprises a cover sheet for covering the surface of the top sheet,
wherein a circumferential edge region of the cover sheet is joined to the absorbing body, and, in a central region of the cover sheet, a plurality of flaps for fixing the absorbing body to clothing are sectionalized by a sectionalizing line which is constituted by a weakened line or a cut line,
wherein the flaps include a front flap and a rear flap that are folded in the folding regions along the front and rear edges of the absorbing body and are expanded in the back-and-forth direction, and a left flap and a right flap that are folded in the folding regions along the left and right side edges of the absorbing body and are expanded in the right-and-left direction,
wherein, at the time of use, the flaps are folded in the folding regions and are expanded so as to be fixed to the clothing, and
wherein a leakage-preventing region is formed on the cover sheet between the folding regions and the circumferential edge region.

2. The absorbent article according to claim 1, wherein the end portions of the sectionalizing line extend without reaching the circumferential edge region of the cover sheet to thereby form the leakage-preventing region on the cover sheet between the folding regions and the circumferential edge region, the leakage-preventing region continuing and expanding over the entire circumference of the cover sheet.

3. The absorbent article according to claim 1, wherein the sectionalizing line is formed so as to curve the ends of the front flap and the rear flap that are folded in the folding regions so as to be fixed to the inner surface of the clothing.

4. The absorbent article according to claim 1, wherein fixing portions for fixing the flaps to the clothing are attached to the flaps, respectively, and flap regions surrounding the fixing portions have a rigidity greater than the rigidity of the remaining flap regions.

5. The absorbent article according to claim 1, wherein the fixing portions for fixing the flaps to the clothing are attached to the flaps being separated away from the sectionalizing line.

6. The absorbent article according to claim 1, wherein the cover sheet is fluid-impermeable.
